# EUROPEAN PATENT SPECIFICATION

(11) **EP 0 637 222 B1**
(45) Date of publication and mention of the grant of the patent: **07.07.1999**
(21) Application number: 93911863.4
(22) Date of filing: 22.04.1993
(51) Int. Cl.: A61B 5/103

(54) **BALANCE PERFORMANCE MONITOR**
VORRICHTUNG ZUR DARSTELLUNG DER GEWICHTSVERTEILUNG
DISPOSITIF POUR EVALUER LA CAPACITE A GARDER L'EQUILIBRE

(30) Priority: 25.04.1992 GB 9209025
(43) Date of publication of application: 08.02.1995
(73) Proprietor: SMS SANDLAND MANUFACTURING SERVICES LIMITED, Harlow, Essex CM19 5TL (GB)
(72) Inventor: WENMAN, Alan, Dunmow, Essex CM6 1UQ (GB); DAUGHTERY, Christopher, Bishops Stortford, Herts CM23 2PB (GB)
(74) Representative: Gillam, Francis Cyril
(86) International application number: GB9300847
(87) International publication number: WO9321825

(56) References cited:
- DE-A- 2 723 775
- DE-A- 3 301 864
- US-A- 4 195 643
- US-A- 4 598 717
- US-A- 4 738 269
- PROC. IEEE NATIONAL AEROSPACE AND ELECTRONICS CONF. vol. 2/2, 20 May 1985, DAYTON (US) pages 1006 - 1011 C.A. PHILLIPS ET AL. 'A Cognitive Feedback System: Approach toward the Total Neural Prosthesis' Section IV. Elements of a Cognitive Feedback Sysytem
- ELECTRONIQUE APLLICATIONS no. 33, December 1983, EVRY (FR) pages 27 - 37 J.-M. JULLIAN 'Analyse dynamique d'empreintes sur APPLE II'

## Description

This invention relates to apparatus for monitoring the balance performance of a user suitable for use for example in physiotherapy as well as in other medical circumstances, in sports both to assist recovery from an injury and to improve performance, and in other fields.

It has been established that the posture and gait of a person can often be improved by monitoring the load carried by each foot, and then displaying essentially in real time those loads to the person in such a way that he may himself adjust the loads until they become substantially the same. In effect, a closed-loop system is set up allowing a user to observe and correct primarily his balance and perhaps also his posture. It is found that if a person suffers for example from disease of the joints or from post-injury trauma, the loads carried by the two feet may differ greatly, but by allowing a person to monitor those loads himself in the manner described above, rapid improvements in the condition from which the user is suffering may be obtained.

A balance performance monitor for humans is known, and comprises a pair of foot-plates upon which a user may stands with one foot on each foot-plate respectively, each foot-plate having an indication of the general position over which a user's foot should be placed and having means to detect the load imparted thereto by the user, and means to display the load imparted to each foot-plate. The user can use such known apparatus to adjust his posture and in this way attempt to make the loads imparted to the two foot-plates substantially equal.

One example of apparatus of the above kind is to be found in DE-A-2723775 and GB-A-1528305. This employs two single-axis foot plates and so can detect the total load imparted by each foot, or if the patient turns through 90°, the front-to-back load on each foot. Experience of using such equipment shows that a mis-diagnosis of true balance performance is quite likely, and even the possibility of assessing there is no clinical problem when in fact there is a significant deficiency in balance performance.

Following extended research into balance and posture, it has now been determined that further and more rapid improvements are possible if the user is supplied with even more information concerning the loading on his two feet, and specifically concerning the front-to-back loading and the side-to-side loading, separately but simultaneously for each foot. Thus, the present invention provides apparatus to monitor the balance performance of a user of the kind described above, characterised in that:
a) the load-detecting means associated with each foot-plate is arranged to detect the load imparted to the respective foot-plate about a first axis lying generally along the length of a user's foot correctly placed with respect to the foot position indication and simultaneously to detect the load imparted to the foot-plate about a second axis generally at right angles to the first axis; and
b) the display means displays in a graphical manner, for each foot-plate separately, the position of the detected loads with respect to a representation of the user's feet.

The load-detecting means associated with each foot-plate could take a variety of forms, but for many users it may be particularly important that the foot-plate has the smallest possible vertical height above a supporting surface, to minimise the vertical lift a user has to impart to his foot, in order to stand on the foot-plate. This may be achieved by having the load-detecting means of each foot-plate in the form of a plurality of load cells disposed beneath the foot-plate, each load cell providing an electrical output dependent upon the load applied thereto. Preferably, however, each foot-plate is supported by a pair of resilient beams provided with strain detectors (gauges) which permit an assessment of the loading on the foot-plate, both in the front-to-back and side-to-side directions. Thus, each foot-plate may be provided with load-detecting means which are disposed to detect the loading substantially at the foot corners of a rectangle which encloses the area of the foot-plate upon which a user should stand. In effect, two load detecting means may be provided at the front of the foot-plate, disposed one to each side thereof, and two load-detecting means at the back of the foot-plate again with one to each side of the foot-plate. Alternatively, the load-detecting means may be arranged at the points of a diamond, with two such means on the front-to-back axis and two on the side-to-side axis. In either case, the foot-plate may conveniently be marked to show the approximate position where a user's foot should be placed and the orientation thereof.

In order that a user may gain optimum benefit from apparatus of this invention, it is important that a user may readily associate his balance performance with the display thereof. To this end, the display means may include a pictorial representation of both feet, each such representation including two series of indicators extending respectively along and transverse to the representation, and which indicators are successively activated dependent upon the output of the load-detecting means. Where there are representations of both feet, then there may be a further series of indicators arranged to represent a comparison of the load imparted by a user to each of the two foot-plates. In this way, a user may monitor the relative loading on his two feet, as well as the front-to-back and side-to-side loading of each foot.

As an alternative, the graphical display may be computer generated and be displayed on a visual display unit. In such a case, various techniques may be employed to show relative loadings, such as the use of colour or different intensities of colour; other techniques will readily suggest themselves to those skilled in the art.

The outputs from the load-detecting devices of the two foot-plates should be suitably processed to yield signals suitable for driving the display means. In a case where each load-detecting device comprises four load cells or strain detectors, it would be possible to combine the individual load cell or detector outputs by a hard-wired controller circuit, though it is convenient to employ a computer to combine the outputs in the appropriate ways. In this case, the analogue outputs from the load cells or detectors may be converted to digital signals for processing by the computer, the computer then driving the display means.

Where a computer is employed to process the data derived from the load-detecting means of each foot-plate, the computer may be used to yield other valuable data to assist an assessment of balance performance. For example, apart from driving a real-time display of balance for viewing by a user an assessment of balance performance and sway co-efficient may be made and displayed. Data may be stored from a period of use of the apparatus by one user, and then further processed for instance to show average loadings. Also, response to certain stimuli - such as if the user is pushed in some direction - may be monitored and analysed, as may the performance of a user on performing some task, such as swinging a golf club, a racket or the like. A computer may also be used to provide an interface to a printer, for giving a permanent record of balance performance data.

This invention also extends to a method of providing a user with a real-time indication of his balance performance using apparatus having a pair of foot-plates upon which the user may stand with one foot on each foot-plate respectively, each foot-plate having an indication of the general position over which a user's foot should be placed and having means to detect the load imparted thereto by the user, and means to display the load imparted to each foot-plate, which method is characterised by simultaneously but separately detecting the front-to-back loading and the side-to-side loadings imparted by each foot of a user on each foot-plate respectively, and processing the detected loadings to drive a graphical display device illustrating to the user in a graphical manner the position of the detected loads with respect to a representation of the user's feet.

By way of example only, one specific embodiment of balance performance monitoring apparatus constructed and arranged in accordance with the present invention will now be described in detail, reference being made to the accompanying drawings, in which:-
Figure 1 is a block diagram of a complete balance performance monitoring apparatus of this invention;
Figure 2 is a vertical sectional view of a foot-plate used in apparatus of Figure 1;
Figure 3 is a side view of the foot-plate of Figure 1;
Figure 4 is a vertical section through the foot-plate of Figures 2 and 3, on an enlarged scale; and
Figures 5 and 6 show alternative display devices for use in the apparatus of Figure 1.

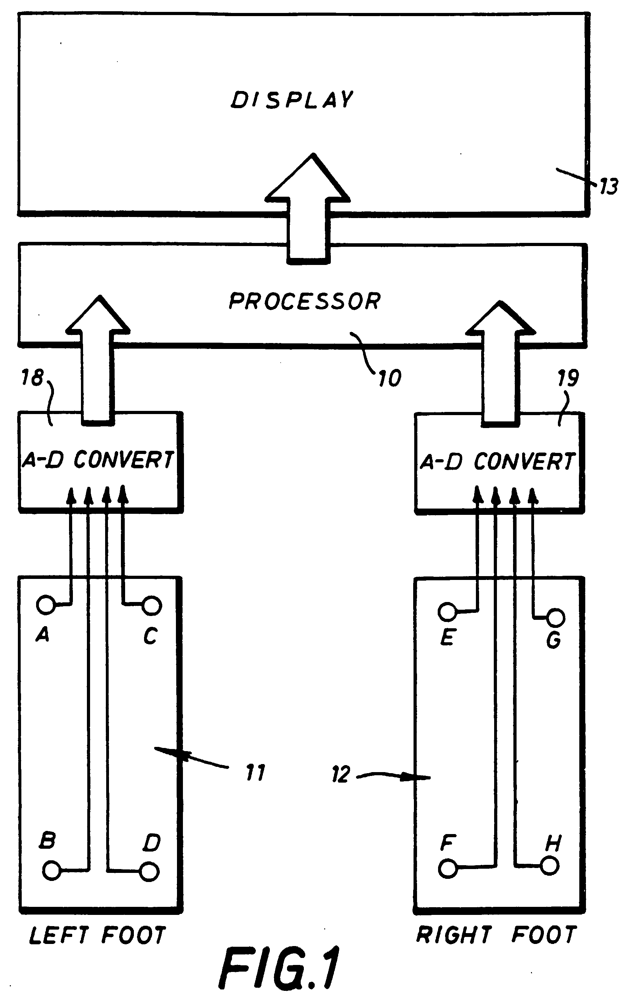

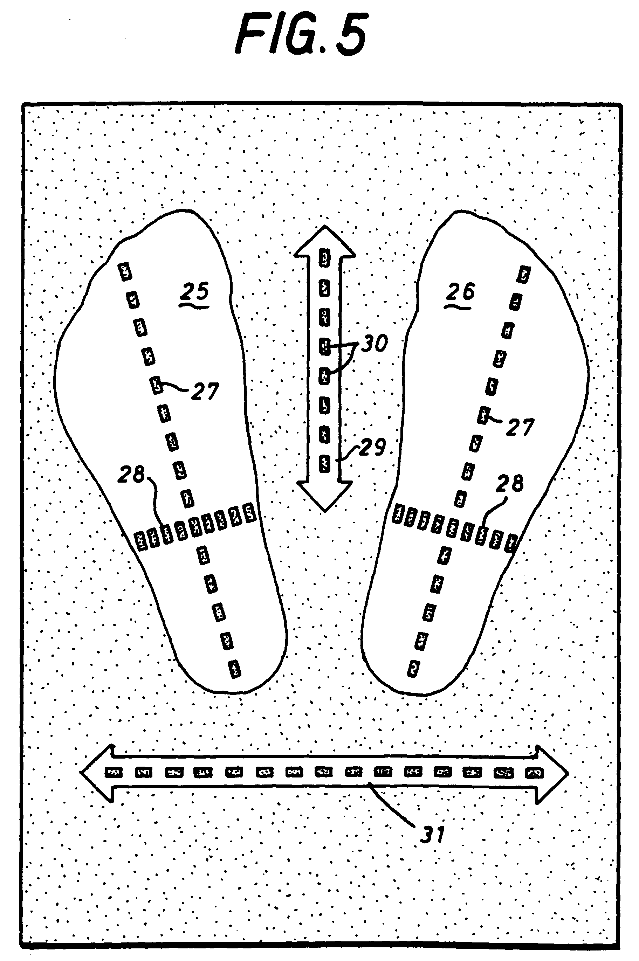

Referring initially to Figure 1, there is shown a block diagram of balance performance monitoring apparatus of this invention, which apparatus employs a computer 10 running an appropriate programme to process data derived from load-measuring points A, B, C and D, and E, F, G and H, of two foot-plates 11 and 12 respectively. The computer 10 is arranged to drive a display device 13, which may take the form of a specially configured display, such as is shown in Figure 5, or may more generally take the form of a video display unit (VDU) driven by the computer so as graphically to show the processed data.

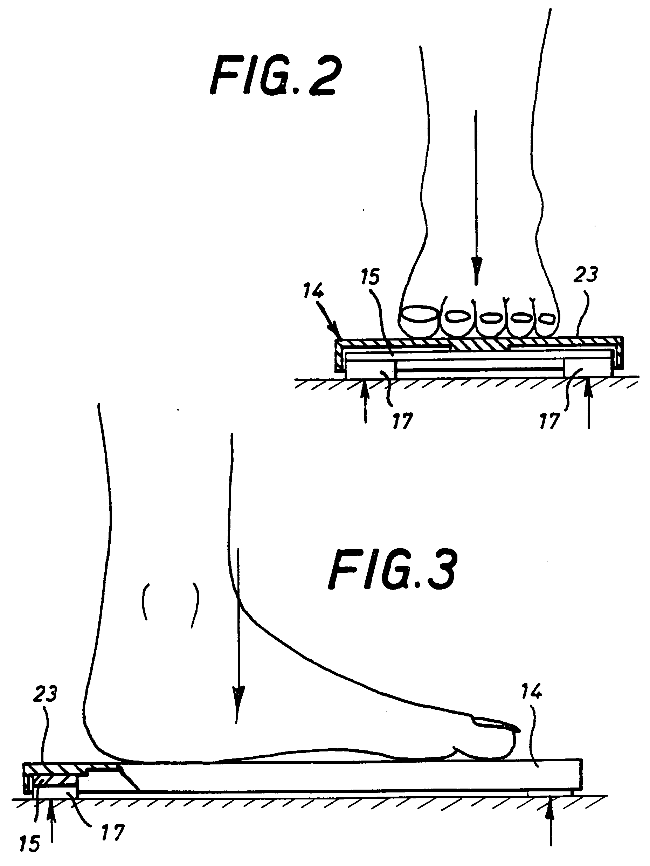

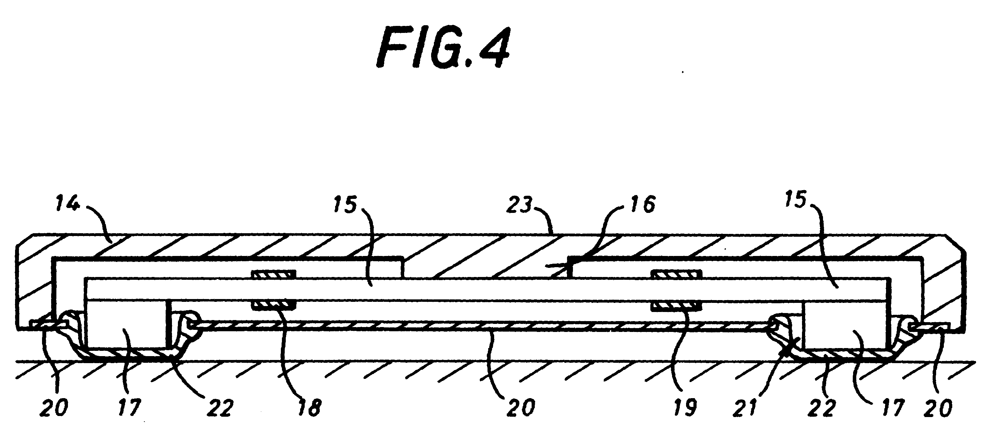

Each foot-plate 11 and 12, shown in more detail in Figures 2, 3 and 4, comprises a rigid plate 14 of a rectangular shape, and of a size slightly greater than the largest human foot likely to be encountered. Arranged at each end of the foot-plate is a transverse resilient beam 15, rigidly attached to a downwardly-projecting boss 16 formed in the underside of the foot-plate. The free ends of each beam are provided with legs 17, and pairs of strain gauges 18 and 19 are mounted on the beams, to each side of the boss 16. A coverplate 20 is attached to the underside of the plate 14, which coverplate has apertures 21 to receive the legs 17. Flexible sealing boots 22 are fitted in the apertures 21 to extend over the legs 17. It is preferred for the upper surface of each plate 14 to carry a diagrammatic representation of a foot, arranged centrally on that plate 14, so that a user will naturally tend to place his foot symmetrically on the plate, as shown in Figures 2 and 3, rather than to one side or to one end of the plate.

The outputs obtained from each strain gauge of a pair are combined to give four outputs from points A to D of plate 11 and E to H of the plate 12, indicative of the loading at those points. These outputs are supplied to respective analogue digital converters 18 and 19, the digital outputs of which are then fed to the computer 10, for processing therewithin. The programme on which the computer runs is arranged to allow a display of the relative loads carried by the left and right feet, as well as the side-to-side weight distribution for each foot, independently, and the front to back weight distribution, again for each foot independently.

In order to allow a display of the balance between the two feet, the computer processes and displays the comparison of:
(A + B + C + D) : (E + F + G + H)

In order to allow a display of the side-to-side weight distribution within the left foot, the computer processes and displays the comparison of:
(A + B) : (C + D)

For the right foot, the computer processes and displays the comparison of:
(E + F) : (G + H)

In a similar way, the computer allows the display of the front to rear weight distribution, independently for the left and right feet by processing and displaying the results of the comparisons:
(A + C) : (B + D) - for the left foot; and
(E + G) : (F + H) - for the right foot.

A typical screen display for illustrating the results of the above comparisons is shown in Figure 5, comprising the front panel of an adjustable stand which panel is printed with diagrammatic representations of the left and right feet 25 and 26. Mounted behind the panel are, for each foot representation, light emitting diodes arranged in two lines 27 and 28, line 27 extending generally centrally along the length of the foot representation, and line 28 transversely thereto. The two lines of light emitting diodes intersect in the region of the approximate centre of pressure of a "normal" person. The display further includes a fore and aft arrow 29 arranged between the representations of the two feet, within which arrow extends a further line 30 of light emitting diodes. Yet another arrow 31 is arranged on the display apparatus to extend from left-to-right between representations of the two feet, there being a line of light emitting diodes within that line.

When the overall apparatus is in use in conjunction with the display device of Figure 5, the appropriate light emitting diode may be illuminated by the computer 10, in order to show how the weight of a user is distributed over his two feet. Moreover, the light emitting diodes within the arrow 31 may appropriately be illuminated to show how the weight is distributed between the two feet of the user. Arrow 29 would not ordinarily be employed when the apparatus is being used just with two foot-plates; it may be used when a seat is connected to the computer 10, to show weight distribution on that seat, in conjunction with arrow 31. Such a seat (not shown) may have four sensors arranged somewhat similarly to those of each foot-plate, and the computer would assess left-to-right balance and front-to-rear balance of a person sitting on that seat, and then display the results of the assessments on the display device.

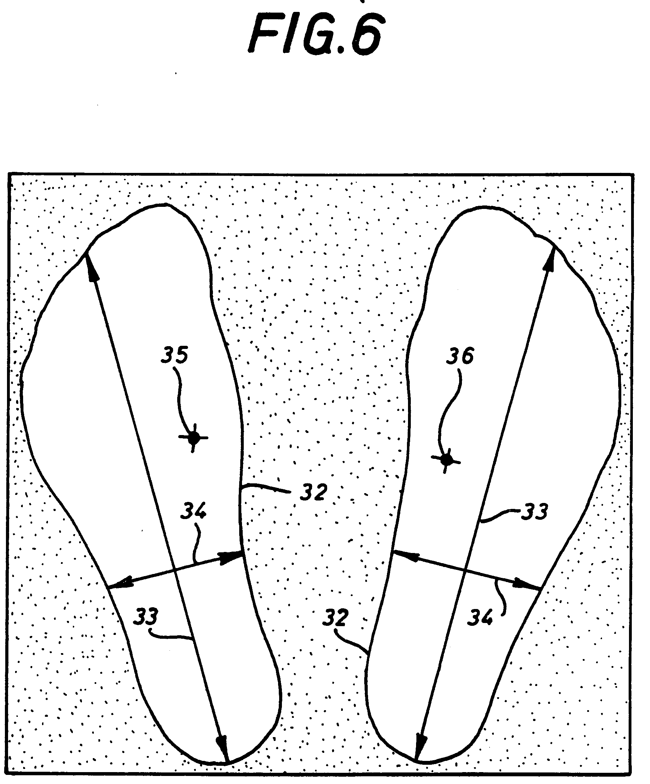

Figure 6 shows an alternative display, such as may be generated by a computer driving a VDU screen. The computer would produce diagrammatic representations 32 of the two feet together with two datum axes 33 and 34, the points of intersection of which being at the centre of pressure referred to above, for a "normal" person. The computer may also generate markers 35 and 36 to show where the user actually has his centres of pressure, for each of his two feet.

By viewing either of the two screen displays described above, when using the apparatus, a person may adjust his posture and observe the effect of that adjustment; in this way, a person may seek to improve his posture so as to achieve a centre of pressure more closely matched to the ideal position. A trained operator (clinician) may also use the apparatus, for an assessment of the subtalar (ankle) joint position, and for knee assessment and an analysis of abnormal gait. The apparatus may further be used for re-training, for example following soft tissue injury to the ankle, which tends to be repetitive in many patients. For this, one foot-plate may be used, with the patient balancing on one foot, on that foot-plate, and leaning rapidly and effectively to correct any deviations from the correct balance.

## Claims

1. Apparatus to monitor the balance performance of a user, which apparatus comprises a pair of foot-plates (11,12) upon which the user may stand with one foot on each foot-plate respectively, each foot-plate (11,12) having an indication of the general position over which a user's foot should be placed and having means to detect the load imparted thereto by the user, and means to display the load imparted to each foot-plate, characterised in that:
a) the load-detecting means (15,18,19) associated with each foot-plate is arranged to detect the load imparted to the respective foot-plate (11,12) about a first axis lying generally along the length of a user's foot correctly placed with respect to the foot position indication and simultaneously to detect the load imparted to the foot-plate about a second axis generally at right angles to the first axis; and
b) the display means (13) displays in a graphical manner, for each foot-plate (11,12) separately, the position of the detected loads with respect to a representation of the user's feet.

2. Apparatus according to Claim 1, wherein each foot-plate (11,12) is supported by a pair of spaced resilient beams (15), provided with strain detectors (18,19) which provide outputs depending upon the loading on the foot-plate.

3. Apparatus according to Claim 1, wherein the load-detecting means of each foot-plate (11,12) comprises a plurality of load cells disposed beneath the foot-plate, each load cell providing an electrical output dependent upon the load applied thereto.

4. Apparatus according to Claim 2 or Claim 3, wherein each foot-plate (11,12) is provided with load-detecting means (18,19) arranged to detect loading substantially at the four corners of a rectangle which encloses the area of the foot-plate upon which a user should stand.

5. Apparatus according to Claim 2 or Claim 3, wherein each foot-plate (11,12) is provided with load-detecting means (18,19) arranged to detect loading at the points of a diamond, with two points on the first axis and two on the second axis of the foot-plate.

6. Apparatus according to any of the preceding claims, wherein the display means (13) includes pictorial representations (25,26) of two feet together with two series of indicators (27,28) for each respectively, extending respectively along and transverse to the representation, and which indicators (27,28) are successively activated dependent upon the outputs of the load-detecting means.

7. Apparatus according to Claim 6, wherein there is a further series of indicators arranged to represent a comparison of the total load imparted by a user to each of the two foot-plates.

8. Apparatus according to any of Claims 1 to 5, wherein the graphical display is computer-generated and is displayed on a visual display unit.

9. Apparatus according to any of the preceding claims, wherein there is additionally provided a seat having load-detecting means arranged to permit an assessment of the left-to-right balance and the front-to-rear balance of a person sitting on that seat, the outputs from the seat additionally being supplied to said display means for display thereon.

10. Apparatus according to any of the preceding claims, wherein the outputs from the load-detecting means of the two foot-plates are supplied to computer apparatus programmed to combine the outputs from the load-detecting means in the appropriate ways.

11. A method of providing a user with a real-time indication of his balance performance using apparatus having a pair of foot-plates (11,12) upon which the user may stand with one foot on each foot-plate respectively, each foot-plate having an indication of the general position over which a user's foot should be placed and having means to detect the load imparted thereto by the user, and means to display the load imparted to each foot-plate, which method is characterised by simultaneously but separately detecting the front-to-back loading and the side-to-side loading imparted by each foot of a user on each foot-plate (11,12) respectively, and processing the detected loadings to drive a graphical display device (13) illustrating to the user in a graphical manner the position of the detected loads with respect to a representation of the user's feet.

## Patentansprüche

1. Vorrichtung zur Darstellung des Gleichgewichtsverhaltens von einem Benutzer, wobei die Vorrichtung ein Paar Fußplatten (11, 12), auf denen der Benutzer mit jeweils einem Fuß auf jeder Fußplatte stehen kann, wobei jede Fußplatte (11, 12) eine Markierung der allgemeinen Position aufweist, auf der ein Fuß des Benutzers plaziert werden sollte, und eine Einrichtung, um die Last zu erfassen, die durch den Benutzer darauf aufgebracht wird, und eine Einrichtung enthält, um die Last anzuzeigen, die auf jede Fußplatte aufgebracht wird, dadurch gekennzeichnet, daß:
a) die Lasterfassungseinrichtung (15, 18, 19), die mit jeder Fußplatte in Beziehung steht, dazu ausgestaltet ist, um die Last zu erfassen, die auf die jeweilige Fußplatte (11, 12) entlang einer ersten Achse aufgebracht wird, die allgemein entlang der Länge eines Fußes des Benutzers verläuft, der bezüglich der Fußpositionsmarkierung korrekt plaziert ist, und um gleichzeitig die Last zu erfassen, die entlang einer zweiten Achse auf die Fußplatte aufgebracht wird, die allgemein im rechten Winkel zu der ersten Achse verläuft; und
b) die Anzeigeeinrichtung (13) auf eine graphische Weise, für jede Fußplatte (11, 12) getrennt, die Position der erfaßten Lasten relativ zu einer Darstellung des Fußes des Benutzers anzeigt.

2. Vorrichtung nach Anspruch 1, bei der jede Fußplatte (11, 12) durch ein Paar von beabstandeten, elastischen Trägern (15) abstützend gehalten ist, die mit Dehnungsdetektoren (18, 19) versehen sind, die abhängig von der Last auf der Fußplatte Ausgaben zur Verfügung zu stellen.

3. Vorrichtung nach Anspruch 1, bei der die Lasterfassungseinrichtung von jeder Fußplatte (11, 12) eine Anzahl von Kraftmeßdosen aufweist, die unter der Fußplatte angeordnet sind, wobei jede Kraftmeßdose in Abhängigkeit von der darauf aufgebrachten Last eine elektrische Ausgabe zur Verfügung stellt.

4. Vorrichtung nach Anspruch 2 oder Anspruch 3, bei der jede Fußplatte (11, 12) mit einer Lasterfassungseinrichtung (18, 19) versehen ist, die dazu ausgestaltet ist, um eine Last im wesentlichen an den vier Ecken eines Rechtecks zu erfassen, das die Fläche der Fußplatte umschließt, auf der ein Benutzer stehen sollte.

5. Vorrichtung nach Anspruch 2 oder Anspruch 3, bei der jede Fußplatte (11, 12) mit einer Lasterfassungseinrichtung (18, 19) versehen ist, die dazu ausgestaltet ist, um eine Last an den Spitzen eines Rhombus zu erfassen, wobei zwei Spitzen auf der ersten Achse und zwei Spitzen auf der zweiten Achse der Fußplatte liegen.

6. Vorrichtung nach einem der vorhergehenden Ansprüche, bei der die Anzeigeeinrichtung (13) bildliche Darstellungen (25, 26) der beiden Füße zusammen mit jeweils zwei Reihen von Markierungen (27, 28) enthält, die sich jeweils entlang und quer zu der Darstellung erstrecken, und die Markierungen (27, 28) abhängig von den Ausgaben der Lasterfassungseinrichtungen nacheinander aktiviert werden.

7. Vorrichtung nach Anspruch 6, bei der eine weitere Reihe von Markierungen vorgesehen ist, die dazu ausgestaltet sind, um einen Vergleich der gesamten Last darzustellen, die durch einen Benutzer auf jede der beiden Fußplatten aufgebracht wird.

8. Vorrichtung nach einem der Ansprüche 1 bis 5, bei der die graphische Anzeige per Computer erzeugt und auf einer visuellen Anzeigeeinheit angezeigt wird.

9. Vorrichtung nach einem der vorhergehenden Ansprüche, bei der außerdem ein Sitz vorgesehen ist, der eine Lasterfassungseinrichtung aufweist, die dazu ausgestaltet sind, um eine Bestimmung des links-nach-rechts-Gleichgewichts und des vorne-nach-hinten-Gleichgewichts einer Person zu ermöglichen, die auf dem Sitz sitzt, wobei die Ausgaben von dem Sitz zusätzlich der Anzeigeeinrichtung zur Anzeige darauf zugeführt werden.

10. Vorrichtung nach einem der vorhergehenden Ansprüche, bei der die Ausgaben von den Lasterfassungseinrichtungen der beiden Fußplatten einem Computer zugeführt werden, der programmiert ist, um die Ausgaben von den Lasterfassungseinrichtungen auf geeignete Weise zu verarbeiten.

11. Verfahren, um einem Benutzer mit einer Echtzeit-Anzeige seines Gleichgewichtsverhaltens zu versorgen, wobei eine Vorrichtung verwendet wird, die ein Paar Fußplatten (11, 12), auf denen der Benutzer mit jeweils einem Fuß auf jeder Fußplatte stehen kann, wobei jede Fußplatte eine Markierung der allgemeinen Position aufweist, auf der ein Fuß des Benutzers plaziert werden sollte, und eine Einrichtung, um die Last zu erfassen, die durch den Benutzer darauf aufgebracht wird, und eine Einrichtung enthält, um die Last anzuzeigen, die auf jede Fußplatte aufgebracht wird, wobei das Verfahren gekennzeichnet ist durch gleichzeitiges, aber separates Erfassen der vorne-nach-hinten-Belastung und der Seite-zur-Seite-Belastung, die durch jeden Fuß eines Benutzers jeweils auf jede Fußplatte (11, 12) aufgebracht wird, und Verarbeiten der erfaßten Belastungen, um eine graphische Anzeigeeinrichtung (13) anzusteuern, durch die dem Benutzer auf graphische Weise die Position der erfaßten Belastungen relativ zu einer Darstellung des Fußes des Benutzers angezeigt wird.

## Revendications

1. Appareil pour représenter le mode d'équilibre d'un utilisateur, cet appareil comprenant une paire de semelles (11, 12) sur lesquelles l'utilisateur peut se tenir debout avec un pied sur chaque semelle respectivement, chaque semelle (11, 12) possédant une indication de la position générale sur laquelle un pied de l'utilisateur doit être placé et possédant des moyens pour détecter la charge qui lui est impartie par l'utilisateur, et des moyens pour afficher la charge impartie à chaque semelle, caractérisé en ce que :
a) les moyens de détection de charge (15, 18, 19) associés à chaque semelle sont agencés pour détecter la charge impartie à la semelle respective (11, 12) autour d'un premier axe s'étendant d'une manière générale selon la longueur du pied de l'utilisateur placé correctement par rapport à l'indication de position du pied et simultanément pour détecter la charge impartie à la semelle autour d'un second axe s'étendant d'une manière générale à angle droit du premier axe; et
b) les moyens d'affichage (13) affichent d'une manière graphique, pour chaque semelle (11, 12) séparément, la position des charges détectées par rapport à une représentation des pieds de l'utilisateur.

2. Appareil selon la revendication 1, dans lequel chaque semelle (11, 12) est supportée par une paire de poutres élastiques (15) espacées, munies de détecteurs de déformation (18, 19), qui fournissent des sorties qui sont fonction du chargement sur la semelle.

3. Appareil selon la revendication 1, dans lequel les moyens de détection de charge de chaque semelle (11, 12) comprennent plusieurs cellules de chargement disposées sous la semelle, chaque cellule de chargement fournissant une sortie électrique qui est fonction de la charge qui lui est appliquée.

4. Appareil selon la revendication 2 ou 3, dans lequel chaque semelle (11, 12) est équipée de moyens de détection de charge (18, 19) agencés pour détecter le chargement sensiblement aux quatre coins d'un rectangle qui entoure la zone de la semelle sur laquelle un utilisateur est censé se tenir debout.

5. Appareil selon la revendication 2 ou 3, dans lequel chaque semelle (11, 12) est équipée de moyens de détection de charge (18, 19) agencés pour détecter le chargement aux sommets d'un losange, avec deux sommets sur le premier axe et deux sur le second axe de la semelle.

6. Appareil selon l'une quelconque des revendications précédentes, dans lequel les moyens d'affichage (13) comprennent des représentations picturales (25, 26) de deux pieds avec deux séries d'indicateurs (27, 28) pour chacune respectivement, s'étendant respectivement le long de la représentation et transversalement à celle-ci, ces indicateurs (27, 28) étant successivement activés en fonction des sorties des moyens de détection de charge.

7. Appareil selon la revendication 6, dans lequel il y a une autre série d'indicateurs agencée pour représenter une comparaison de la charge totale impartie par un utilisateur à chacune des deux semelles.

8. Appareil selon l'une quelconque des revendications 1 à 5, dans lequel l'affichage graphique est généré par ordinateur et affiché sur une unité d'affichage visuel.

9. Appareil selon l'une quelconque des revendications précédentes, dans lequel il est en outre prévu un siège possédant des moyens détecteurs de charge agencés pour permettre une évaluation de l'équilibre gauche-droite et de l'équilibre avant-arrière d'une personne assise sur ce siège, les sorties du siège étant en outre envoyées aux moyens d'affichage pour affichage sur ceux-ci.

10. Appareil selon l'une quelconque des revendications précédentes, dans lequel les sorties des moyens détecteurs de charges des deux semelles sont envoyés à de l'appareillage informatique programmé pour combiner convenablement les sorties des moyens détecteurs de charge.

11. Procédé pour fournir à un utilisateur une indication en temps réel de son mode d'équilibre en utilisant un appareil possédant une paire de semelles (11, 12) sur lesquelles l'utilisateur peut se tenir debout avec un pied sur chaque semelle respectivement, chaque semelle possédant une indication de la position générale sur laquelle un pied de l'utilisateur doit être placé, et possédant des moyens pour détecter la charge qui lui est impartie par l'utilisateur, et des moyens pour afficher la charge impartie à chaque semelle, le procédé étant caractérisé en ce qu'on détecte simultanément mais séparément le chargement avant-arrière et le chargement d'un côté et de l'autre impartis par chaque pied d'un utilisateur sur chaque semelle (11, 12) respectivement, et en ce qu'on traite les chargements détectés pour piloter un dispositif d'affichage graphique illustrant d'une manière graphique pour l'utilisateur la position des charges détectées par rapport à une représentation des pieds de l'utilisateur.
